(19) **Europäisches Patentamt / European Patent Office / Office européen des brevets**

(11) **EP 3 708 953 B1**

(12) **EUROPEAN PATENT SPECIFICATION**

(45) Date of publication and mention of the grant of the patent:
**22.12.2021 Bulletin 2021/51**

(51) Int Cl.:
**G01C 21/06** *(2006.01)*    **G01C 21/08** *(2006.01)*
**G01C 21/14** *(2006.01)*    **G01C 21/16** *(2006.01)*
**G01C 22/00** *(2006.01)*    **A61B 5/11** *(2006.01)*

(21) Application number: **19163315.5**

(22) Date of filing: **15.03.2019**

(54) **INERTIAL POSITIONING AND TRACKING METHOD AND SYSTEM WITH INNOVATIVE FUNCTIONALITY FOR MAGNETOMETER AUTO-CALIBRATION AND FOR ANGULAR DRIFT COMPENSATION/CORRECTION**

INERTIALPOSITIONIERUNGS- UND -VERFOLGUNGSVERFAHREN UND -SYSTEM MIT NEUARTIGER FUNKTIONALITÄT ZUR MAGNETOMETERAUTOKALIBRIERUNG UND ZUR WINKELVERSATZKOMPENSIERUNG/-KORREKTUR

PROCÉDÉ ET SYSTÈME DE POSITIONNEMENT INERTIEL ET DE SUIVI AVEC FONCTION INNOVANTE D'ÉTALONNAGE AUTOMATIQUE DE MAGNÉTOMÈTRE ET DE COMPENSATION/CORRECTION DE LA DÉRIVE ANGULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**16.09.2020 Bulletin 2020/38**

(73) Proprietor: **Dune S.r.l.**
**00183 Roma (IT)**

(72) Inventors:
• **DE MARINIS, Enrico**
**00183 Roma (IT)**

• **ULIANA, Michele**
**00198 Roma (IT)**

(74) Representative: **Sordini, Lorenzo et al**
**Studio Torta S.p.A.**
**Via Viotti, 9**
**10121 Torino (IT)**

(56) References cited:
**IT-A1-201700 088 521**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 708 953 B1

**Description**

## CROSS-REFERENCE TO RELATED APPLICATIONS

This patent application is related to Italian patent application no. 102017000119916 filed on 23/10/2017.

## TECHNICAL FIELD OF INVENTION

The present invention concerns the positioning and tracking of people in environments where, in general, no positioning/navigation service/system is available/utilizable and, in particular, where:

- no satellite positioning/navigation service is available/utilizable, i.e., of the GNSS (Global Navigation Satellite System) type, such as the GPS, GALILEO, or GLONASS systems;
- nor are other infrastructures to aid positioning (such as mobile phone networks, WiFi networks or radio beacons) available/utilizable.

In particular, the present invention concerns an inertial positioning and tracking method and system in which estimates of the position and path of a user are generated on the basis of inertial measurements and magnetic field measurements carried out by a measuring device fixed on a foot of a user to be located and tracked.

## STATE OF THE ART

As is known, very often GNSS signals are unable to penetrate indoor environments, for example, inside buildings or underground areas. Consequently, GNSS signals often cannot be used in these indoor environments for locating and tracking personnel and/or equipment.

In the same way, the positioning of personnel and equipment can be impossible even in tactical environments (for example, military operations or anti-terrorist theatre) where instruments capable of effectively interfering with GNSS signals are used.

Therefore, the need for reliable indoor positioning and tracking technologies is particularly felt in all those operational scenarios where one or more teams of people (for example, firemen, police, special forces, etc.) must coordinate themselves and/or be coordinated (for example, in order to carry out rescue, aid, or anti-terrorism operations, etc.) in indoor environments (for example, inside skyscrapers, underground stations, etc.) where GNSS-based positioning is not available/utilizable.

In recent years, the indoor positioning problem has been tackled by innumerable systems and methods, which can be classified in three main categories:

1) solutions based on purpose-built infrastructures, i.e. that use specially provided devices or instruments deployed in the indoor environment (for example, radio beacons, RFID tags, etc.), or based on dedicated measuring campaigns (for example, geomagnetic field measurements);
2) solutions based on available infrastructures, i.e. that use already existing infrastructures and/or already available information (for example, the so-called WiFi fingerprinting techniques, which are based on mobile telephone network signals, digital maps of the building, etc.);
3) solutions that do not require any external infrastructure, i.e. that exploit only information coming from sensors worn by the person it is intended to locate and track.

An example of a solution belonging to the first category is provided in EP 2 345 293 B1, where an indoor localization system for locating an electronic mobile device within an indoor environment is described.

In particular, the indoor localization system according to EP 2 345 293 B1 comprises:

- a plurality of transmitting nodes arranged in different positions inside the indoor environment and configured to transmit radio frequency (RF) signals; and
- at least one electronic mobile device configured to receive the RF signals transmitted by the transmitting nodes.

The indoor localization system according to EP 2 345 293 B1 is configured to operate:

- in a training mode, wherein the electronic mobile device computes reference quantities based on powers of the RF signals received in different reference positions within the indoor environment; and
- in a locating mode, wherein the electronic mobile device computes current quantities based on powers of the RF signals received in its current location within the indoor environment, wherein the current location of the electronic mobile device is determined based on the current and the reference quantities.

Furthermore, in the indoor localization system according to EP 2 345 293 B1:

- the transmitting nodes transmit the RF signals on at least two different radio channels in both the training and locating modes;
- in the training mode, the electronic mobile device computes, for each reference position and for each of the at least two different radio channels, corresponding reference quantities based on the RF signals received on said radio channel in said reference position; and
- in the locating mode, the electronic mobile device computes, for each of the at least two different radio channels, corresponding current quantities based on the RF signals received on said radio channel in its current position.

Whereas, an example of solution belonging to the second category is provided in US 2017/0026804 A1, where an indoor positioning system is described that correlates positions on a map with respective wireless access point (AP) fingerprints, where each wireless AP fingerprint includes a plurality of wireless AP signal measurements taken at the position on the map to which said fingerprint is correlated. In this way, the indoor positioning system according to US 2017/0026804 A1 generates a map in which the fingerprints of wireless AP signals are georeferenced and is therefore able to determine the position of a user device based on said map and the wireless AP signal measurements taken by said user device at its current position.

In addition, two solutions belonging to the third category are provided in Applicant's Italian patent applications RM2012A000641 and TO2014U000050.

In particular, both RM2012A000641 and TO2014U000050 describe a positioning device that can be worn by a pedestrian operator which comprises:

- an inertial sensor configured to measure accelerations and angular velocities of the pedestrian operator and to output raw data indicative of time series of the measured accelerations and angular velocities;
- a non-linear interpolation filter, which is coupled to the inertial sensor to receive the raw data therefrom and which comprises an artificial neural network trained to filter and interpolate the raw data so as to filter out noise and thermal drift, using weights obtained through automatic learning of human gait features;
- a subsystem for integration of motion equations, which is coupled to the non-linear interpolation filter to receive the filtered and interpolated data therefrom and which is configured to

  - estimate position, velocity and path of the pedestrian operator by processing the filtered and interpolated data, and
  - output corresponding preliminary position, velocity and path estimates; and

- a subsystem for estimation of errors of position and velocity, which is coupled to the subsystem for integration of motion equations to receive the preliminary position, velocity and path estimates therefrom and which is configured to

  - estimate position and velocity errors based on subjective biometric rules that model human gait features and are not produced by measuring instruments, and
  - correct the preliminary position, velocity and path estimates based on the estimated errors, thereby obtaining correct final estimates.

According to RM2012A000641, the artificial neural network comprises:

- a plurality of recurrent multilayer perceptrons with delayed outputs in a multi-stream configuration; and
- an extended Kalman filter;

wherein the various recurrent multilayer perceptrons with delayed outputs are configured to:

- filter and interpolate different time-staggered portions of the raw data by using one and the same set of weights and
- provide corresponding time-staggered outputs indicative of the filtered and interpolated data;

wherein the artificial neural network is configured to time align and combine the time-staggered outputs of the recurrent multilayer perceptrons, thereby obtaining overall filtered and interpolated data indicative of the accelerations and angular velocities of the pedestrian operator at each sampling instant; and wherein the extended Kalman filter is configured to update the same set of weights used by all the recurrent multilayer perceptrons.

Whereas, according to TO2014U000050, the artificial neural network comprises:

- an extended Kalman filter; and
- a plurality of bidirectional systems in a multi-stream configuration based on back-propagation over time, each of which includes a respective causal section and a respective retrocausal section;

wherein the bidirectional systems are configured to:

- filter and interpolate different time-staggered portions of the raw data using one and the same set of weights and
- provide corresponding time-staggered outputs indicative of the filtered and interpolated data;

wherein the artificial neural network is configured to time align and combine the time-staggered outputs of the bidirectional systems, thereby obtaining overall filtered and interpolated data indicative of the accelerations and angular velocities of the pedestrian operator at each sampling instant; and wherein the extended Kalman filter is configured to update the same set of weights used by all the bidirectional systems.

Additionally, a further example of solution belonging to the third category is provided in US 2014/203970 A1, which describes a system and method for estimating the position of an object, for example a person, an animal or a machine. In particular, the system according to US 2014/203970 A1 includes a first and a second inertial measurement unit, a first and a second originator antenna and a first and a second antenna transponder. This system uses data supplied by the inertial measurement units to estimate a position of the object. In addition, the system computes a range measurement between the originator antenna and the first transponder antenna, a first carrier phase difference (CPD) measurement between the second transponder antenna and the first originator antenna and a second CPD measurement between the second originator antenna and the first transponder antenna. The range measurement and at least one CPD measurement are used to update a Kalman filter for estimating the position of the object. Furthermore, the system according to US 2014/203970 A1 also determines updates for the Kalman filter when one of the inertial measurement units is in a zero-velocity condition. Solutions belonging to the first two categories generally provide better performance than solutions belonging to the third category. Unfortunately, however, the real applicability of solutions belonging to the first two categories is limited, as these solutions necessarily require the availability of external infrastructures (mobile telephone networks, wireless networks, RFID tags, etc.) and/or preliminary reference measurements.

Instead, the currently known solutions belonging to the third category are unable to assure satisfactory accuracy for long times (i.e. hours).

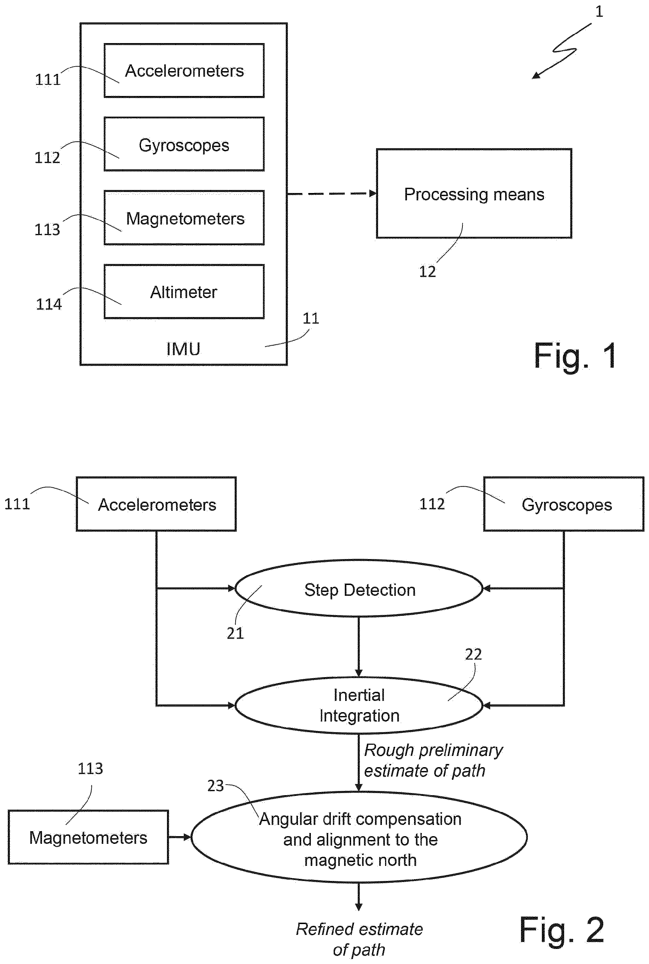

Figure 1 schematically shows a high-level reference architecture of an inertial positioning and tracking system (indicated as a whole by reference numeral 1) of the known type belonging to the third category.

In particular, the inertial positioning and tracking system 1 includes:

- an inertial measurement unit (IMU) 11 that

  - includes

    - accelerometers 111 (typically three or more accelerometers 111 configured to carry out acceleration measurements along three mutually orthogonal spatial directions/axes),
    - gyroscopes 112 (typically three or more gyroscopes 112 configured to detect attitude variations along three mutually orthogonal spatial directions/axes),
    - magnetometers 113 (typically three or more magnetometers 113 configured to carry out magnetic field measurements along three mutually orthogonal spatial directions/axes)
    - and, if necessary, also an altimeter 114, and

  - is generally integrated in a device wearable by a person to be located and tracked (typically, the device inside which the IMU 11 is integrated is fixed, in use, on a foot of a user, for example on a shoe, on the ankle, etc.); and

- processing means 12 configured to receive the data recorded by the IMU 11 and to process the received data so as to locate and track the user wearing the IMU 11.

Figure 2 schematically shows (via a functional block diagram) the positioning and tracking logic implemented, in use, by the system 1.

In particular, as shown in Figure 2, the processing carried out by the processing means 12 includes the following steps:

4

a) step detection (block 21) based on the data supplied by the accelerometers 111 and the gyroscopes 112, on the basis of which metrics are constructed to decide what are the moments in time when the foot touches the ground while walking about;

b) inertial integration (block 22) of the data supplied by the accelerometers 111 and the gyroscopes 112 and the information obtained through step a) (block 21), to obtain a rough preliminary estimate of the path followed by the user wearing the IMU 11; and

c) compensation of the angular drift and alignment of the path to the magnetic north (block 23) on the basis of the data supplied by the magnetometers 113.

In particular, in said step c) the data supplied by the magnetometers 113 is used to correct/compensate the angular drift that distorts the path estimated with just the inertial data (i.e. the data supplied by the accelerometers 111 and gyroscopes 112), thus obtaining a refined estimate of the path followed by the user wearing the IMU 11. Typically, compensation of the angular drift and alignment of the path to the magnetic north (block 23) serve to ensure that different users can share a same absolute topographical reference (in fact, if M users were to be simultaneously tracked without any alignment to the north, the respective estimated paths would be rotated by "arbitrary" angles with respect to one another, because an effective absolute reference would actually be lacking).

More specifically, the use of magnetometer data (i.e. data supplied by the magnetometers 113) is commonly used to provide a correction to the angular drift in the horizontal plane. In fact, the magnetometers 113 with which the IMU 11 is equipped, if properly calibrated, provide the indication of the magnetic north and this information enables compensating the angular drift that distorts the path estimated with just the inertial data (i.e. the data supplied by the accelerometers 111 and gyroscopes 112).

Unfortunately, however, the currently known techniques of angular drift compensation and alignment to the magnetic north (block 23) necessarily require that precise calibration of the magnetometers 113 be carried out before a mission begins, otherwise the information supplied by said magnetometers 113 would be virtually unusable. In particular, the lack of calibration of magnetometers 113 introduces additional errors that result in path estimates that are less accurate than those obtained without magnetometer correction.

The calibration of magnetometers 113 that must be carried out before starting each mission can be implemented by using different known methodologies, such as, for example, those described in:

- Yan Xia Liu, Xi Sheng Li, Xiao Juan Zhang and Yi Bo Feng, "Novel Calibration Algorithm for a Three-Axis Strapdown Magnetometer", Sensors 2014, 14(5), pages 8485-8504, DOI: 10.3390/s140508485; or
- Vitali Andrea, "Ellipsoid or sphere fitting for sensor calibration", ST Microelectronics, DT0059 Design Tip, DT 0059 Rev 2, August 2016.

In any case, the currently known magnetometer calibration methodologies, although different in terms of the mathematical techniques employed, are typically united by the following operating characteristics:

1) the magnetometer calibration must be carried out in an environment with little (or zero) magnetic interference;
2) before a mission is started, a user must move the magnetometers 113 (i.e. the IMU 11) by performing a series of specific movements, for example circular or figure-of-eight paths, or random movements on a spherical cover, etc.;
3) the raw data produced by the magnetometers 113 while carrying out the aforementioned specific movements must be processed to obtain the estimates of certain calibration parameters.

These operating characteristics can of course be satisfied when carrying out experiments in the laboratory (where it is possible to set up an environment to achieve limited magnetic interference). Unfortunately, however, magnetometer calibration in a real operating environment is effectively unfeasible, because:

- a real operating environment is, in general, magnetically polluted (consider, for example, an urban scenario) and, in any case, the level of magnetic pollution is unknown and therefore it is not possible to have a sufficient level of confidence on the effective quality of the calibration;
- it may be impossible for an operator to carry out the aforementioned specific movements with the magnetometers 113 (i.e. with the IMU 11); consider, for example, any user who must carry out a mission in which time has a fundamental role for the safety of lives or security of goods (for example, firemen, police forces, etc.).

From these observations, it can be deduced that the calibration of magnetometers in a real operating environment cannot actually be carried out before the start of a mission.

**OBJECT AND SUMMARY OF THE INVENTION**

A general object of the present invention is to provide a methodology of magnetometer auto-calibration and angular drift compensation/correction for inertial positioning and tracking applications, this methodology enabling to carry out angular drift compensation and alignment to the magnetic north in real operating environments.

Furthermore, a specific object of the present invention is to provide a methodology of magnetometer auto-calibration and angular drift compensation/correction that:

- is robust with regards to magnetic pollution in the operating environment; and
- does not impose any magnetometer calibration procedure to be carried out by an operator, neither before a mission or after it has started.

These and other objects are achieved by the present invention in so far as it relates to an inertial positioning and tracking method and system, according to what is defined in the appended claims.

**BRIEF DESCRIPTION OF DRAWINGS**

For a better understanding of the present invention, some preferred embodiments, provided by way of non-limitative example, will now be described with reference to the accompanying drawings (not to scale), in which:

- Figure 1 schematically shows an inertial positioning and tracking system of a known type;
- Figure 2 schematically shows a positioning and tracking logic implemented by the inertial positioning and tracking system shown in Figure 1;
- Figure 3 schematically shows an inertial positioning and tracking system according to a preferred embodiment of the present invention; and
- Figures 4-9 show examples of experimental data obtained by the applicant in relation to a magnetometer auto-calibration and angular drift compensation/correction functionality according to the present invention.

**DETAILED DESCRIPTION OF PREFERRED EMBODIMENTS OF THE INVENTION**

The following description is provided to enable an expert in the field to embody and use the invention. Various modifications to the embodiments shown will be immediately obvious to experts and the generic principles described herein could be applied to other embodiments and applications without departing from the scope of the present invention as defined in the appended claims.

Thus, the present invention is not intended to be limited to the embodiments set forth herein, but is to be accorded the widest scope consistent with the principles and features disclosed herein and defined in the appended claims.

In general, the present invention relates to an inertial positioning and tracking method that comprises fixing, on a foot of a user to be located and tracked, a measuring device configured to carry out inertial measurements and magnetic field measurements, wherein the inertial measurements include acceleration measurements and attitude variation measurements.

Furthermore, the inertial positioning and tracking method according to the present invention also comprises carry out the following operations through processing means:

- generating rough estimates of position and path of the user on the basis of the inertial measurements carried out by the measuring device; and
- refining the rough estimates of position and path of the user by carrying out an angular drift compensation/correction based on the magnetic field measurements carried out by the measuring device.

In particular, generating rough estimates of position and path of the user includes:

- determining user's step characteristics on the basis of the inertial measurements carried out by the measuring device; and
- generating the rough estimates of position and path of the user on the basis of the inertial measurements carried out by the measuring device and of the user's step characteristics determined.

Furthermore, determining user's step characteristics includes detecting halt time intervals, each related to a corresponding step of the user.

Furthermore, generating the rough estimates of position and path of the user includes determining rotation matrices between a first and a second reference system, wherein said first reference system is integral with the Earth and said second reference system is integral with the measuring device.

According to the present invention, the step of refining the rough estimates of position and path of the user includes:

- for each step of the user, storing respective average values

  - of the magnetic field measurements carried out by the measuring device in the corresponding halt time interval related to said step of the user, and
  - of the rotation matrices determined for the corresponding halt time interval related to said step of the user;

- estimating magnetic calibration parameters (or bias) for the last N steps of the user, where N is an integer greater than one (i.e. $N > 1$; preferably, $100 \le N \le 1000$);
- estimating values of angular drift and orientation with respect to the magnetic north for the last N steps of the user based on the magnetic calibration parameters estimated for said last N steps of the user; and
- correcting the rough estimates of position and path of the user for the last N steps of said user on the basis of the values of angular drift and orientation with respect to the magnetic north estimated for said last N steps of the user.

In particular, the step of estimating magnetic calibration parameters for the last N steps of the user includes:

- defining magnetic calibration metrics related to the last N steps of the user; and
- applying a predefined multivariate minimization technique to the magnetic calibration metrics related to the last N steps of the user, thereby finding magnetic calibration parameters that minimize said metrics.

In greater detail, defining magnetic calibration metrics related to the last N steps of the user includes defining:

- calibrated magnetic field values in the second reference system related to the last N steps of the user based on

  - the average values of the magnetic field measurements stored for each of said last N steps of the user and
  - magnetic calibration parameters;

- calibrated magnetic field values in the first reference system related to the last N steps of the user based on

  - the average values of the rotation matrices stored for each of said last N steps of the user and
  - the calibrated magnetic field values in the second reference system related to said last N steps of the user;

- calibrated magnetic heading angles related to the last N steps of the user on the basis of the calibrated magnetic field values in the first reference system related to said last N steps of the user; and
- a difference function indicative of a difference between magnetic heading and a corresponding angular drift.

Furthermore, applying a predefined multivariate minimization technique to the magnetic calibration metrics related to the last N steps of the user includes performing said predefined multivariate minimization technique so as to find values of the magnetic calibration parameters that minimize a variance of the difference function.

Preferably, the calibrated magnetic field values in the second reference system related to the last N steps of the user differ from the average values of the magnetic field measurements stored for said last N steps of the user by the magnetic calibration parameters, which are constant additive coefficients.

Conveniently, the steps of estimating magnetic calibration parameters for the last N steps of the user, estimating values of angular drift and orientation with respect to the magnetic north for the last N steps of the user and correcting the rough estimates of position and path of the user for the last N steps of said user are carried out every P steps of the user, where P is an integer greater than zero and less than N (i.e. $0 < P < N$; for example, P=10, assuming a value N=1000 has been adopted) .

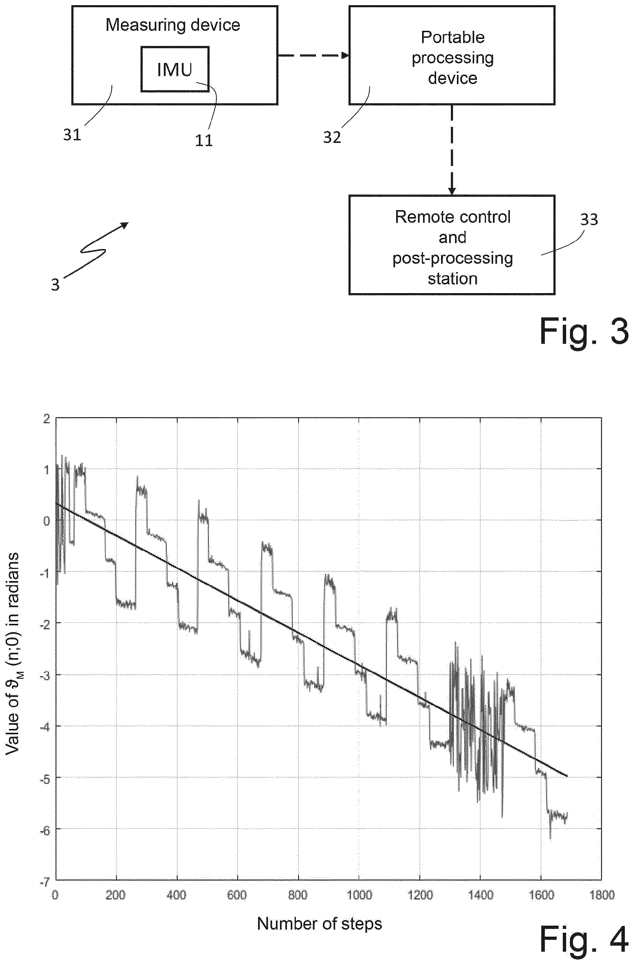

Figure 3 schematically shows an inertial positioning and tracking system (indicated as a whole by reference numeral 3) according to a preferred (but absolutely non-binding, nor even limitative) embodiment of the present invention.

In particular, the inertial positioning and tracking system 3 includes:

- a measuring device 31 designed to

- be fixed on a foot of a user (for example a fireman) to be located and tracked (for example, said measuring device 31 can be conveniently configured to be tied around a shoe or to an ankle of a user),
- carry out inertial measurements (preferably, acceleration measurements and attitude variation measurements over time (i.e. angular velocity) along three predefined, mutually orthogonal, spatial directions/axes, i.e. three-dimensional measurements of acceleration and attitude variations),
- carry out magnetic field measurements (preferably along the three predefined, mutually orthogonal, spatial directions/axes, i.e. three-dimensional magnetic field measurements), and
- output data/signals indicative of the inertial measurements and the magnetic field measurements carried out;

- a portable processing device 32 (for example a smartphone/tablet opportunely programmed by means of a special inertial positioning and tracking software application (a so-called app), or a portable, pocket electronic device equipped with wireless communication means and opportunely programmed by means of special software/firmware code for inertial positioning and tracking), said portable processing device 32 being destined to be transported, in use, by the user to be located and tracked (for example, inside a trouser or jacket pocket of said user) to carry out "local" real-time processing; in particular, said portable processing device 32 is configured to

  - communicate, in a wired or wireless fashion (for example, via Bluetooth technology), with the measuring device 31 fixed on the foot of said user to receive, from said measuring device 31, data/signals indicative of the inertial measurements and the magnetic field measurements carried out by said measuring device 31,
  - generate rough estimates of position and path of the user based on the inertial measurements carried out by the measuring device 31, and
  - refine (i.e. correct) the rough estimates of position and path of the user on the basis of the magnetic field measurements carried out by the measuring device 31 (and, if necessary, in the case of a portable processing device 32 equipped with a GNSS receiver, also the GNSS signals, if available and reliable), thereby generating refined estimates of position and path of the user; and

- a remote control and post-processing station 33 (for example, a fixed or portable computer (i.e. a laptop or a server), which is configured to

  - remotely communicate (for example, via one or more mobile telephone networks, one or more wireless networks, one or more networks based on Internet Protocol (IP), etc.) with the portable processing device 32 to receive, from said portable processing device 32, data/signals indicative of the refined estimates of position and path of the user, and
  - carry out a further refinement of the refined estimates of position and path of the user via post-processing techniques (for example, of a known type).

Preferably, the measuring device 31 internally integrates the IMU 11 of the inertial positioning and tracking system 1 shown in Figure 1 and previously described.

Furthermore, and again preferably, the portable processing device 32 is programmed to implement in real time the positioning and tracking logic shown in Figure 2 and previously described. In particular, said portable processing device 32 is preferably programmed to carry out:

a) the step detection (block 21 in Figure 2), i.e. determining user's step characteristics based on the inertial measurements carried out by the measuring device 31 (in particular, on the basis of the data supplied by the accelerometers 111 and the gyroscopes 112 of the IMU 11);
b) the inertial integration (block 22 in Figure 2) of the inertial measurements carried out by the measuring device 31 (in particular, the data supplied by the accelerometers 111 and the gyroscopes 112 of the IMU 11) and the user's step characteristics determined in step a) (block 21 in Figure 2) to obtain a rough preliminary estimate of the path followed by the user; and
c) the compensation of the angular drift and alignment to the magnetic north (block 23 in Figure 2) based on the magnetic field measurements carried out by the measuring device 31 (in particular, on the basis of the data supplied by the magnetometers 113 of the IMU 11) to obtain a refined estimate of the path followed by the user.

Preferably, the portable processing device 32 is programmed to carry out the step a) of step detection (block 21 in Figure 2) by implementing the innovative functionality of detecting conditions of halt and stillness of a user's foot described in Applicant's Italian patent application 102017000088521 .

At this point, it is extremely important to underline the fact that the inertial positioning and tracking system 3 is provided purely by way of non-limitative example, it being understood that the present invention could be suitably implemented in many other alternative forms; for example:

- the portable processing device 32 could be suitably configured to also carry out the functions performed by the remote control and post-processing station 33 (which, in this case, might not be present); or
- the portable processing device 32 might not be present and the related functions could be performed directly by the remote control and post-processing station 33 (for example, for indoor environment mapping applications where no real-time processing of the data provided by the IMU 11 is required in order to locate and track users); or
- the portable processing device 32 could be integrated together with the measuring device 31 in a single wearable device;
- etc.

In the following description, the group of magnetometers integrated in the measuring device 31 (preferably, the magnetometers 113 of the IMU 11) shall be referred to, for simplicity of description and in an absolutely clear manner for an expert in the field, as "triaxial magnetometer" (as it performs three-dimensional magnetic field measurements, in particular along three mutually orthogonal spatial directions/axes) or also "magnetometer sensor".

The present invention concerns the aforementioned step c) of compensation of the angular drift and alignment to the magnetic north (block 23 in Figure 2).

In particular, the present invention concerns a new and innovative method of magnetometer auto-calibration and angular drift compensation/correction that enable estimating calibration parameters (hereinafter also referred to as "calibration biases") during the mission, refining the values in proportion to the distance travelled by the tracked operator.

In particular, the method of magnetometer auto-calibration and angular drift compensation/correction according to a preferred embodiment of the present invention includes:

c1) a data collection step, wherein, at each step of the user, the following are stored in a buffer (i.e. in a buffer memory), preferably of the First-In First-Out (FIFO) type, of length N (as previously stated, with N being an integer greater than one, i.e. $N > 1$; preferably, $100 \leq N \leq 1000$) :

- the values of the magnetic field measurements carried out by the measuring device 31 (in particular, by the related triaxial magnetometer) and
- a rotation matrix from a first reference system integral with the Earth (for example, a Cartesian three-dimensional reference system having a predefined orientation with respect to the cardinal points) to a second reference system integral with the measuring device 31 (hereinafter, said first reference system integral with the Earth and said second reference system integral with the measuring device 31 will be referred to, in a manner unequivocally known to an expert in the field, as "navigation frame" and "body frame", respectively);

c2) a step of combined estimation of the calibration biases, the angular drift and the magnetic north, wherein on the basis of the data collected in step c1), every P steps (as previously stated, with P being an integer greater than zero and less than N, i.e. $0 < P < N$; for example P=10), an estimation is carried out of the calibration biases of the triaxial magnetometer through the construction of metrics and a multivariate minimization algorithm; and

c3) a step of correction of the path estimated in step b) (block 22 in Figure 2), wherein, every P steps, the estimated path is backwards corrected for N steps, based on the estimates of the angular drift and the magnetic north obtained in step c2).

In order to describe the steps c1)-c3) of the method of magnetometer auto-calibration and angular drift compensation/correction according to the present invention in greater detail, hereinafter the following mathematical notation will be used in the description:

$t_c$ sampling time of signals of the IMU 11;

$M_u(n)$ average value supplied by the triaxial magnetometer at the $n^{th}$ step for axis $u$;

$M_u(t)$ value supplied by the triaxial magnetometer at time t for axis u;

$\mathbf{m}_b(t)$ magnetic vector $[M_x(t), M_y(t), M_z(t)]$ in the body frame at time $t$;

$\mathbf{m}_b(n)$ average magnetic vector in the body frame at the $n^{th}$ step; $\mathbf{m}_n(n)$ average magnetic vector in the navigation frame at the $n^{th}$ step;

$\mathbf{m}_n(n; \mathbf{q})$ average magnetic vector in the navigation frame at the $n^{th}$ step, relative to the $\mathbf{q}^{th}$ magnetic bias hypothesis applied to the body frame;

$k(n)t_c$ first time value when $n^{th}$ step starts;

$D(n)$ number of temporal samples over which the $n^{th}$ step extends; $\mathbf{R}_n^b(n)$

average value of the rotation matrix from the body frame to the navigation frame for the $n^{th}$ step;

$\mathbf{R}_n^b(t)$ rotation matrix at time $t$;

$\vartheta_M(n)$ average value of the magnetic heading in the navigation frame for the $n^{th}$ step;

$\vartheta_M(n; \mathbf{q})$ average value of the magnetic heading in the navigation frame for the $n^{th}$ step, assuming $\mathbf{q}$ as the magnetic bias vector hypothesis;

$\vartheta_{MC}(n; \mathbf{q})$ magnetic heading at step $n$, assuming $\mathbf{q}$ as the magnetic biases vector in the body frame, after compensation of the angular drift $\delta\omega(\mathbf{q})$;

$\mathbf{q}$ $\mathbf{q} = [q_x, q_y, q_z]^T$ calibration biases vector on the three axes;

W diagonal matrix of the estimated covariance on the estimates of $d(n;\mathbf{q})$;

$\delta\Psi_N(\mathbf{q})$ path realignment angle to the magnetic north, assuming q as the magnetic biases vector in the body frame;

$\delta\omega(\mathbf{q})$ estimated angular drift assuming q as the magnetic biases vector in the body frame.

The various steps c1)-c3) of the method of magnetometer auto-calibration and angular drift compensation/correction will be described below in detail according to said preferred embodiment of the present invention.

**c1) Data collection**

The first step c1) of data collection serves to build a memory that, starting from the current step, goes backwards for N steps, storing the necessary data for processing in the next step c2), where the magnetic calibration parameters are estimated.

Mathematically, the term "$n^{th}$ step" means the set of contiguous temporal samples that, in the step a) of step detection (block 21 in Figure 2), have been identified as related to a halt condition. In particular, "halt condition" (or also simply "halt") is meant to be those moments in time when, while the user is walking about, the user's foot completely rests on the ground between one step and the next, and therefore has zero velocity in all directions.

At the $n^{th}$ step, the three magnetic field values are averaged, for each axis and for the entire duration of the foot's halt, according to the relation

$$M_u(n) = \frac{1}{D(n)} \sum_{m=0}^{D(n)-1} M_u((k(n) + m)t_c) \qquad (1)$$

where

- $M_u(n)$ is the average value supplied by the triaxial magnetometer at the $n^{th}$ step, for axis $u$;
- $M_u(t)$ is the value supplied by the triaxial magnetometer at time t, for axis u;
- $k(n)t_c$ is the first time value when the $n^{th}$ step starts; and
- $D(n)$ is the number of temporal samples over which the $n^{th}$ step extends.

The rotation matrices (3x3 in size), estimated in the step b) of inertial integration (block 22 in Figure 2), are used to obtain an average rotation matrix during the halt. From the formal standpoint, this average value (Riemann matrix average) is given by

$$\mathbf{R}_n^b(n) = \left( \prod_{m=0}^{D(n)-1} \mathbf{R}_n^b((k(n) + m)t_c) \right)^{\frac{1}{D(n)}} \qquad (2)$$

which has an extremely high computational weight, given that a step can even extend over several tens of temporal samples. However, taking into account that small variations in the rotation matrix are expected during the foot's halt, it is more than justified to expect small variations between $\mathbf{R}_n^b(mt_c)$ and $\mathbf{R}_n^b((m + 1)t_c)$ , and so the following can be written

$$\mathbf{R}_n^b(mt_c)\mathbf{R}_n^b((m+1)t_c) \cong \left(\mathbf{R}_n^b(mt_c)\right)^2 (I + \boldsymbol{\Delta}),$$

where $\Delta$ is antisymmetric (with elements $|\delta_{n,k}|<<1$; $\delta_{k,k}=0$) and I is the identity matrix. By way of example, the average rotation between two matrices $\mathbf{R}_1$ and $\mathbf{R}_2$, which differ by $\Delta$, would be

$$\mathbf{R}_m = (\mathbf{R}_1\mathbf{R}_2)^{\frac{1}{2}} \cong \left(\mathbf{R}_1\left(\mathbf{R}_1(\mathbf{I}+\boldsymbol{\Delta})\right)\right)^{\frac{1}{2}} = \left(\mathbf{R}_1^2(\mathbf{I}+\boldsymbol{\Delta})\right)^{\frac{1}{2}} \cong \mathbf{R}_1\left(\mathbf{I}+\frac{\boldsymbol{\Delta}}{2}\right) \cong \frac{1}{2}(\mathbf{R}_1 + \mathbf{R}_2).$$

These considerations thus allow opting for a less rigorous relation, but justified by the small attitude variations that occur during the foot's halt:

$$\mathbf{R}_n^b(n) = \frac{1}{D(n)} \sum_{m=0}^{D(n)-1} \mathbf{R}_n^b((k(n)+m)t_c) \tag{3}$$

where

- $\mathbf{R}_n^b(n)$ is the average value of the rotation matrix for the $n^{th}$ step; and

- $\mathbf{R}_n^b(t)$ is the value of the rotation matrix at time t.

It is then possible to pass to the computation of the heading value estimated from the magnetometer data. In this regard, it should be noted that the magnetic heading (i.e. the angle that defines the direction of the magnetic north in the navigation frame) should always be zero for a perfectly calibrated system, with the navigation frame aligned to the magnetic north and devoid of angular drift effects. At the $n^{th}$ step, the average magnetic vector in the navigation frame is given by:

$$\mathbf{m}_n(n) = \frac{1}{D(n)} \sum_{m=0}^{D(n)-1} \mathbf{R}_n^b((k(n)+m)t_c)\, \mathbf{m}_b((k(n)+m)t_c) \tag{4}$$

Three FIFO buffers are thus filled in this step and, at generic step n, they will contain the following quantities:

$$\mathbf{M}_u(n) = [M_u(n-N+1), \dots, M_u(n-1), M_u(n)]$$
$$\mathbf{R}(n) = [\mathbf{R}_n^b(n-N+1), \dots, \mathbf{R}_n^b(n-1), \mathbf{R}_n^b(n)] \tag{5}$$

**c2) Combined estimation of the calibration biases, the angular drift and the magnetic north**

In the step c2) it is assumed that the calibrated values differ from the measured ones by the additive coefficients, considered constant over long periods of time with respect to the time of the mission in course, whereby the following can be written:

$$M_u^c(n) = M_u(n) + q_u \tag{6}$$

where

- $M_u^c(n)$ is the value supplied by the triaxial magnetometer calibrated at the $n^{th}$ step, for axis $u$; and
- $q_u$ is the value of the calibration bias for axis $u$ (magnetic bias), which is assumed time-independent within the scope of duration of the operations under examination.

The generic vector of the calibration bias is indicated as $\mathbf{q} = [q_x, q_y, q_z]^T$.

The magnetic vector in the navigation frame is computed as

$$\begin{pmatrix} m_{n,x}(n;\mathbf{q}) \\ m_{n,y}(n;\mathbf{q}) \\ m_{n,z}(n;\mathbf{q}) \end{pmatrix} = \boldsymbol{m}_n(n;\mathbf{q}) = \mathbf{R}_n^b(n)(\boldsymbol{m}_b(n) + \mathbf{q}) \qquad (7)$$

In complex notation, the resultant magnetic heading (assuming **q** as the magnetic or magnetometer bias) can be computed as:

$$\vartheta_M(n;\mathbf{q}) = \mathtt{atan2}\big(m_{n,y}(n;\mathbf{q}), m_{n,x}(n;\mathbf{q})\big) \qquad (8)$$

The magnetic heading (suffering from angular drift and polarization due to uncompensated biases) can then be estimated for each step and each magnetic bias hypothesis.

For each possible value q of the magnetometer biases, the angular drift $\delta\omega(\mathbf{q})$ can be estimated as the angular coefficient of a weighted linear regression, in which the weights are proportional to the local variance $\vartheta_M(n;\mathbf{q})$, so as to give less weight to local fluctuations (noise or dynamic spikes). The linear regression values applied to $\vartheta_M(n;\mathbf{q})$ are the angular drift $\delta\omega(\mathbf{q})$ and the difference between the magnetic north and inertial north, known as $\delta\psi_N(\mathbf{q})$. Their values are provided by the relation:

$$\begin{bmatrix} \delta\psi_N(\mathbf{q}) \\ \delta\omega(\mathbf{q}) \end{bmatrix} = (\mathbf{X}^T\mathbf{W}\mathbf{X})^{-1}\mathbf{X}^T\mathbf{W}\boldsymbol{\vartheta}_M(\mathbf{q}) \qquad (9)$$

where

$$\mathbf{X}^T = \begin{bmatrix} 1, 1, \dots, 1 \\ 1, 2, \dots, N \end{bmatrix}$$
$$\boldsymbol{\vartheta}_M(\mathbf{q}) = [\vartheta_M(1;\mathbf{q}), \vartheta_M(2;\mathbf{q}), \dots, \vartheta_M(N;\mathbf{q})] \qquad (10)$$

and where **W** is the diagonal matrix of the covariance estimated on the estimates of $\vartheta_M(n;\mathbf{q})$.

With regard to this, Figure 4 shows an example, taken from experimental data, of the trend of $\vartheta_M(n;\mathbf{q})$ as a function of the steps trodden; furthermore, Figure 4 also shows the linear regression obtained from relation (9.

It should be noted that, independently of the value of N, the inversion in (9 always involves the use of a 2×2 matrix and is therefore not particularly onerous; the greatest computational effort regards the (minimum) 6N operations to be performed, with products and sums. Alternatively, a sub-optimal approach with less computational effort could also be adopted, in which the estimates of the offset with respect to the magnetic north and of the angular drift can be obtained from the relations:

$$\delta\psi_N(\mathbf{q}) = \frac{1}{N}\sum_{n=1}^{N} d(n;\mathbf{q}) \qquad (11)$$

$$\delta\omega(\mathbf{q}) = \frac{2}{N}\left(\sum_{n=1}^{N/2} d(n;\mathbf{q}) - \sum_{n=\frac{N}{2}+1}^{N} d(n;\mathbf{q})\right) \qquad (12)$$

At this point, the angular drift estimated from relation (9 or (12 is used to compensate $\vartheta_M(n;\mathbf{q})$ with the estimated drift, according to the relation:

$$\vartheta_{M_C}(n;\mathbf{q}) = \vartheta_M(n;\mathbf{q}) - \delta\omega(\mathbf{q})(n-1) \qquad (13)$$

Ideally, for a perfectly calibrated magnetometer sensor and a perfect estimate of angular drift, relation (13 would provide a constant value, equal to the offset with respect to the magnetic north.

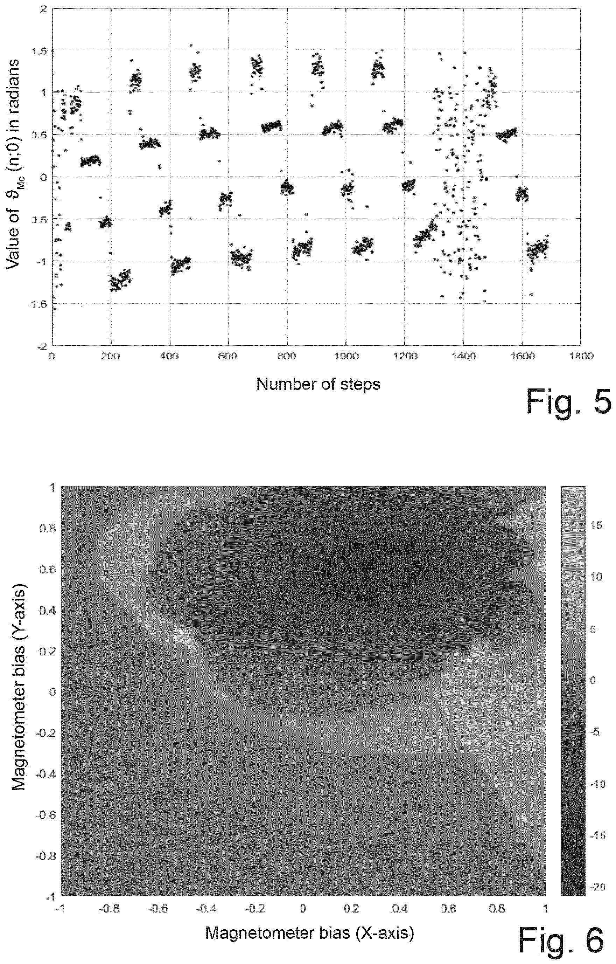

With regard to this, Figure 5 shows an example, taken from actual experimental data, representing the trend of the relation (13, where the linear drift component has been removed.

From the relation (13, the average and the variance of all N samples available in the buffer is estimated, according to the relations:

$$\mu_d(\mathbf{q}) = \frac{1}{N}\sum_{n=1}^{N}\vartheta_{M_C}(n;\mathbf{q}) \qquad (14)$$

$$\sigma_d^2(\mathbf{q}) = \frac{1}{N-1}\sum_{n=1}^{N}\left(\vartheta_{M_C}(n;\mathbf{q}) - \mu_d(\mathbf{q})\right)^2 \qquad (15)$$

With regard to this, Figure 6 shows an example, taken from real experimental data, representing the trend of the metrics (15 for a set of physically possible values for the magnetometer biases on X and Y axes (actually, the algorithm varies the magnetometer biases on the three axes of measurement X, Y and Z and the angular drift of the path, and so a function of four instead of two variables should have been shown); the absolute minimum value of the metrics, which should indicate the real bias values, can be observed in Figure 6.

The most probable value $\hat{\mathbf{q}}$ for the estimate of the magnetometer biases $\mathbf{q}$ is the one that minimizes the variance estimated in (15, conditioned by the maximum and minimum variability limits physically (or constructively) possible for the components of $\mathbf{q}$, represented by a vector $\mathbf{b}$. This condition can be formally expressed by

$$\hat{\mathbf{q}} = \underset{\{|\mathbf{q}|\leq\mathbf{b}\}}{\arg\min}\,\sigma_d^2(\mathbf{q}) \qquad (16)$$

Relation (16 is a problem of constrained multivariate minimization and can be efficiently solved with numerous known methods present in the available literature (such as, for example, that described by M. Bazaraa, H. Sherali and C. Shetty in "Nonlinear Programming, Theory and Applications", J. Wiley & Sons, New York, third edition, 2006), although it should be noted that the problem has a small number of variables to be estimated (the three components of the magnetic bias) and that even sub-optimal estimates can be acceptable, as within the ambit of other errors that weigh on the inertial tracking system, achieving the optimal best for (16 might not be the best compromise between tracking result accuracy and computational complexity. In this perspective, an acceptable solution for (16 could also be obtained through a classical exhaustive search of the entire solution space, subdividing the possible solution space (limited by the limit vector b) into a discrete grid of Q elements; for each single grid value, referred to as $\mathbf{q}_k$, $\sigma_d^2(\mathbf{q}_k)$ is calculated via (15 and then the $\mathbf{q}_k$ with which the absolute minimum is reached is chosen.

The corresponding values of the angular drift $\delta\omega)(\hat{\mathbf{q}})$ from (9 or from (12 and the difference of the magnetic north $\delta_{\psi_N}(\hat{\mathbf{q}})$ from (9 or from (11 are then calculated for the value estimated for $\hat{\mathbf{q}}$ from the solution of (16.

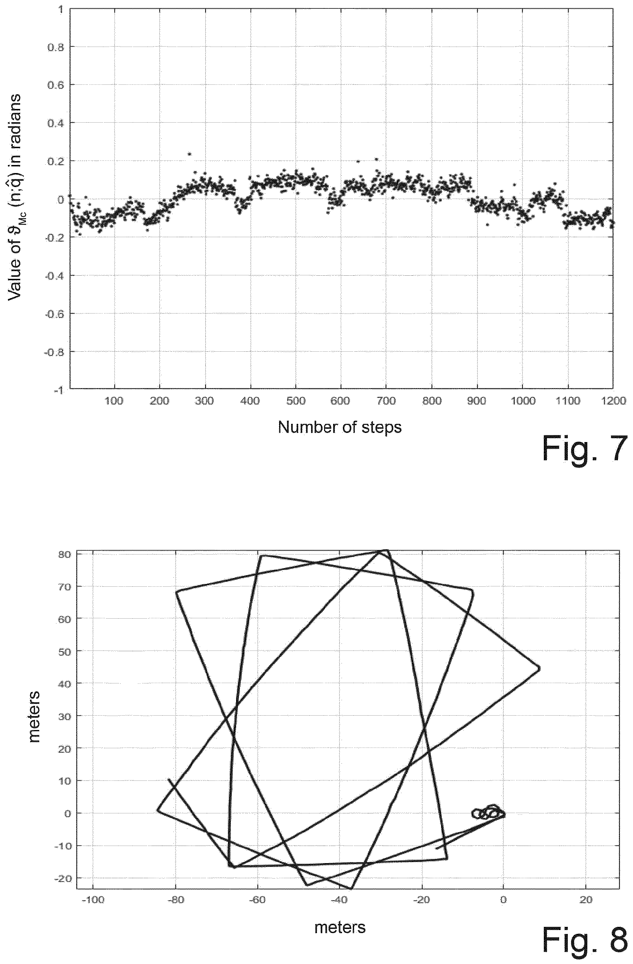

Figure 7 shows an example, taken from experimental data, representing the trend of the relation (13, in which the value of the magnetometer biases estimated in (16 was used.

### c3) Correction of the estimated path

Once problem (16 has been solved, the path estimated in the step b) (block 22 in Figure 2) is backwards corrected for N steps, by applying the following recursive relation

$$\begin{aligned}\alpha(n) &= \alpha(n-1) + \delta\omega(\hat{\mathbf{q}})\\ \mathbf{R}(n) &= \begin{pmatrix} \cos\alpha(n) & \sin\alpha(n)\\ -\sin\alpha(n) & \cos\alpha(n) \end{pmatrix}\\ \mathbf{p}(n) &= \mathbf{p}(n-1) + \mathbf{R}(n)(\mathbf{p_0}(n) - \mathbf{p_0}(n-1))\end{aligned} \qquad (17)$$

where the components of the vector $\mathbf{p}_0(n) = [x_0(n), y_0(n)]^T$ are coordinates of the path on the horizontal plane at step n, without any correction, while $\mathbf{p}(n) = [x(n), y(n)]^T$ are the same coordinates following the correction. Relation (17 runs from 2 to N and is initialized with $\alpha(1) = 0$.

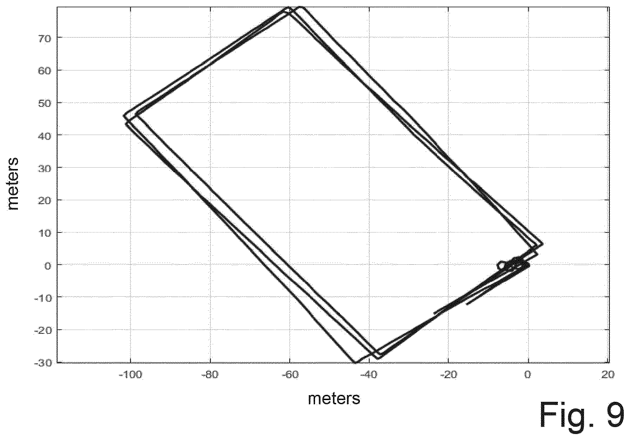

Figures 8 and 9 show the course of the path followed by a user, respectively without any correction related to the magnetic heading and following correction of the angular drift obtained from (17.

As previously explained, the magnetic calibration parameters are estimated on the basis of the rotation matrices that, in turn, are obtained on the basis of the inertial measurements. In fact, said rotation matrices are obtained from equation (3), which only contains quantities derived from the inertial measurements.

Furthermore, equation (8) enables estimating the magnetic heading affected by angular drift and by polarization due to uncompensated biases.

It is important to note that the magnetic calibration parameters are not exclusively estimated on the basis of the rotation matrices; in fact, the latter, although constituting an element of the estimate, are not the only element necessary to obtain an estimate of said magnetic calibration parameters.

In particular, if data processing stopped at just equation (8), magnetic heading values still affected by the drift would be obtained and they would be useless for correction of the path estimated with just the inertial data, as they would be affected by an error of the same type and entity that it is intended to correct.

Therefore, according to the present invention, other criteria are also introduced and assumed as significant, to achieve a corrected estimate of the magnetic calibration parameters that is virtually error free. More specifically, it is assumed that:

i) the (unknown) magnetic calibration coefficients have a purely additive and time-independent nature within the scope of duration of the operations under examination (equation (6)); this assumption is substantiated in that the magnetometers are actually always pre-compensated for the "soft-iron" component (of a multiplicative nature and which is performed only once and forever), while they cannot be for the "hard-iron" component (of an additive nature and which changes on each switching on of the magnetometer);

ii) the angular drift between magnetic heading and inertial heading is well approximated by a linear regression law, at least for all the N steps before the current one; an assumption quantitatively expressed by equation (9); this assumption is substantiated, by way of example, by the experimental data provided in Figure 4;

iii) the variance of the difference between magnetic heading and inertial heading reaches an absolute minimum value only for the right magnetic bias values (unknown), an assumption quantitatively expressed by equation (16); this assumption is substantiated, by way of example, by the experimental data provided in Figure 6.

Given the aforesaid assumptions i), ii) and iii), the solution of equation (16) provides, at output, optimal values (in the least squares sense) for the magnetic biases (where this characteristic has no counterpart, neither in the prior art in general, nor in US 2014/203970 A1 in particular).

As previously described, for the solution of equation (16), it is possible to resort to a numeric method known in literature or even use a sub-optimal method. In any case, it is important the underline the innovative aspect related precisely to the construction and the significance of equation (16), while its solution can be obtained with various numeric methods known in literature.

The magnetic biases estimated by the solution of equation (16) do not therefore derive exclusively from just the inertial rotation matrices (affected by drift), but it is the assumptions i), ii) and iii) that enable reaching an optimal estimate (in the least squares sense) of the magnetic biases, which are unknown beforehand.

The "optimal" magnetic bias values, obtained by the solution of equation (16), are then used to obtain a corresponding "optimal" value of the drift and the magnetic north through equation (9) and the path estimated on the basis of just the inertial data is backwards corrected through a recursive relation "from the past to the present", provided by equation (17). With regard to this, it is worth noting that also the introduction of equation (17) for the correction of estimated path does not have any correspondence in the prior art.

From a less analytical and therefore less rigorous, but certainly more intuitive standpoint, this profound difference can be understood with qualitative reasoning: in the absence of inertial angular drift and of magnetic bias, the magnetic north estimated by the magnetometer is always set at a fixed angle, independently of the path taken by the user. In the presence of inertial angular drift and of magnetic bias, the magnetic north estimated by the magnetometer will be at an angle that varies with time (due to the inertial angular drift) and will fluctuate as a function of the particular point of the path (due to magnetic biases); equation (16) is formed to answer the question "among all the possible inertial angular drifts and magnetic biases, what are the ones that ensure that the magnetic north is set at a fixed angle independently of time andof the point of the path?".

Therefore, in consideration of what has been previously explained, because of the assumptions i), ii) and iii) and the solution of equation (16), the method according to the present invention ensures that estimation of the magnetic calibration parameters is not affected by an error of the same nature and entity as that which influences the rotation matrices obtained on the basis of the inertial measurements.

Thus, as explained, the magnetometer biases (unknown beforehand) are estimated in an "optimal" manner from equation (16) and this gives a correct estimate of the angular drifts from equation (9), resulting in an estimate of a new path with a sharp drop in drift, through equation (17); moreover, by way of example, this is borne out by a comparison between Figure 8 and Figure 9: the first figure shows the result of a purely inertial path estimate without any correction, while the second figure shows the path obtained using the technique of angular drift compensation/correction according to the present invention. Thus, the comparison between Figure 8 and Figure 9 shows, by means of experimental data, the real operating efficiency of the method according to the present invention in solving the technical problem of magnetometer auto-calibration and angular drift compensation/correction.

Many technical advantages of the present invention are immediately evident from the foregoing description.

In particular, it is important to stress that the present invention enables achieving two main types of advantage, namely an improvement in the tracking performance and an improvement in the use of inertial positioning and tracking systems in real applications.

With regard to the improvement in tracking performance, the present invention has been conceived specifically to improve the performance of the tracking system as a whole. In fact, with the introduction of the invention, the angular drift (which negatively influences the accuracy of the path estimated with purely inertial methods) is significantly lessened, at the same time providing an absolute reference with respect to the magnetic north (and geographic north, by compensating magnetic drift) .

In particular, without the invention, an inertial positioning and tracking system based on IMU with low-cost Micro ElectroMechanical Systems (MEMS) technology would have difficulty in providing performance in line with requirements (for example, 5 m of error) of end users (for example, firemen) for periods exceeding 10 minutes; instead, with the introduction of the invention, the required accuracy can be maintained for periods exceeding 30-45 minutes (depending on the type of path and the level of magnetic pollution in the environment).

Furthermore, with regard to improving the use of inertial positioning and tracking systems in real applications, the present invention has a significant impact on the practical use of a inertial positioning and tracking system; in fact, in a real environment, it may be impossible (due to logistic or time constraints) to have an adequate step dedicated to calibration of the magnetometers prior to the start of operations; in addition, even when sufficient time is hypothetically available for magnetometer calibration, this might be of no use or even detrimental, as the place used for calibration might be magnetically polluted or polarized, resulting in erroneous calibration estimating that, if used to compensate angular drift, would effectively introduce more marked distortion of the estimated path. On the other hand, the methodology introduced by the invention enables avoiding any initial calibration, obtaining gradual calibration during the course of operations that, by averaging the magnetic readings over ample path lengths, mitigates the possible presence of local noise or polarization in the magnetic field.

In conclusion, it is clear that various modifications can be applied to the present invention without departing from the scope of the invention as defined in the appended claims.

**Claims**

1. Inertial positioning and tracking method, comprising fixing, on a foot of a user to be located and tracked, a measuring device (31) configured to carry out inertial measurements and magnetic field measurements, wherein the inertial measurements include acceleration measurements and attitude variation measurements;

   the inertial positioning and tracking method further comprising carrying out the following operations by processing means (32):

   - generating rough estimates of position and path of the user on the basis of the inertial measurements carried out by the measuring device (31); and
   - refining the rough estimates of position and path of the user by carrying out an angular drift compensation/correction based on the magnetic field measurements carried out by the measuring device (31);

   wherein generating rough estimates of position and path of the user includes:

   - determining user's step characteristics on the basis of the inertial measurements carried out by the measuring device (31); and
   - generating the rough estimates of position and path of the user on the basis of the inertial measurements carried out by the measuring device (31) and of the user's step characteristics determined;

wherein determining user's step characteristics includes detecting halt time intervals during which the user's foot completely rests on the ground between successive steps thereby having zero velocity in all directions, whereby each halt time interval is related to a corresponding step of the user;

wherein generating the rough estimates of position and path of the user includes determining rotation matrices between a first and a second reference system, wherein said first reference system is integral with the Earth and said second reference system is integral with the measuring device (31) ;

**characterised in that** refining the rough estimates of position and path of the user includes:

• for each step of the user, storing respective average values of

- the magnetic field measurements carried out by the measuring device (31) in the corresponding halt time interval related to said step of the user, and
- the rotation matrices determined for the corresponding halt time interval related to said step of the user;

• defining magnetic calibration metrics related to the last N steps of the user, wherein N is an integer greater than one, and wherein said magnetic calibration metrics are defined such that

- calibrated magnetic field values in the second reference system related to the last N steps of the user are defined based on the average values of the magnetic field measurements stored for said last N steps of the user and on magnetic calibration parameters related to said last N steps of the user,
- calibrated magnetic field values in the first reference system related to the last N steps of the user are defined based on the average values of the rotation matrices stored for said last N steps of the user and on the calibrated magnetic field values in the second reference system related to said last N steps of the user,
- calibrated magnetic heading angles related to the last N steps of the user are defined based on the calibrated magnetic field values in the first reference system related to said last N steps of the user, and
- a difference function is defined, which is indicative of a difference between magnetic heading and a corresponding angular drift;

• estimating the magnetic calibration parameters related to the last N steps of the user by applying a predefined multivariate minimisation technique to the magnetic calibration metrics related to said last N steps of the user so as to determine values of said magnetic calibration parameters that minimise a variance of the difference function;

• estimating values of angular drift and of orientation with respect to the magnetic north for the last N steps of the user based on the estimated values of the magnetic calibration parameters related to said last N steps of the user; and

• correcting the rough estimates of position and path of the user for the last N steps of said user based on the values of angular drift and orientation with respect to the magnetic north estimated for said last N steps of the user.

2. The inertial positioning and tracking method of claim 1, wherein the calibrated magnetic field values in the second reference system related to the last N steps of the user differ from the average values of the magnetic field measurements stored for said last N steps of the user by the magnetic calibration parameters, which are constant additive coefficients.

3. The inertial positioning and tracking method according to claim 1 or 2, wherein the steps of defining magnetic calibration metrics related to the last N steps of the user, estimating the magnetic calibration parameters related to the last $N$ steps of the user, estimating values of angular drift and of orientation with respect to the magnetic north for the last $N$ steps of the user, and correcting the rough estimates of position and path of the user for the last $N$ steps of said user are carried out every $P$ steps of the user, wherein $P$ is an integer greater than zero and lower than $N$.

4. Inertial positioning and tracking system (3), comprising:

• a measuring device (31), which is designed to be fixed on a foot of a user to be located and tracked, and is configured to carry out inertial measurements and magnetic field measurements, wherein the inertial measurements include acceleration measurements and attitude variation measurements; and

• processing means (32) configured to receive signals/data indicative of the inertial measurements and the magnetic field measurements carried out by the measuring device (31) and to carry out the steps of generating rough estimates of position and path of the user and refining the rough estimates of position and path of the user of the inertial positioning and tracking method as defined in any preceding claim.

5. The inertial positioning and tracking system of claim 4, wherein the processing means are integrated into a portable processing device (32), which is:

• designed to be transported by the user to be located and tracked when said user wears the measuring device (31) on his/her foot; and
• configured to be connected, in a wireless or wired fashion, to said measuring device (31) to receive signals/data indicative of the inertial measurements and the magnetic field measurements carried out by said measuring device (31).

6. The inertial positioning and tracking system of claim 5, wherein the portable processing device (32) is a smartphone, a tablet, or a portable, pocket electronic device.

7. The inertial positioning and tracking system of claim 4, wherein the measuring device (31) and the processing means (32) are integrated into one and the same device.

8. The inertial positioning and tracking system according to any claim 4-7, further comprising a remote control and post-processing station (33) configured to:

• receive signals/data indicative of the refined estimates of position and path of the user generated by the processing means (32); and
• carry out a further refinement of the refined estimates of position and path of the user via one or more predefined post-processing techniques.

9. Computer program product comprising one or more portions of software and/or firmware code loadable on processing means (32) of an inertial positioning and tracking system (3); wherein said inertial positioning and tracking system (3) also comprises a measuring device (31), which is designed to be fixed on a foot of a user to be located and tracked, and is configured to carry out inertial measurements and magnetic field measurements, wherein the inertial measurements include acceleration measurements and attitude variation measurements;

wherein the processing means (32) are configured to receive signals/data indicative of the inertial measurements and the magnetic field measurements carried out by the measuring device (31),
and wherein said portion(s) of software and/or firmware code is/are such as to cause, when loaded on the processing means (32), the latter to become configured to carry out the steps of generating rough estimates of position and path of the user and refining the rough estimates of position and path of the user of the inertial positioning and tracking method as defined in any claim 1-3.

**Patentansprüche**

1. Inertialpositionierungs- und -verfolgungsverfahren, welches die Befestigung einer Messvorrichtung (31) an einem Fuß eines zu ortenden und zu verfolgenden Benutzers umfasst, welche so konfiguriert ist, dass sie Inertialmessungen und Magnetfeldmessungen durchführt, wobei die Inertialmessungen Beschleunigungsmessungen und Messungen der Lageänderung umfassen;

wobei das Inertialpositionierungs- und -verfolgungsverfahren ferner die Durchführung der folgenden Operationen durch Verarbeitungsmittel (32) folgendes umfasst:

- Erzeugen von groben Schätzungen der Position und des Weges des Benutzers auf der Grundlage der von der Messvorrichtung (31) durchgeführten Inertialmessungen; und
- Verfeinerung der groben Schätzungen der Position und des Weges des Benutzers durch die Durchführung einer Kompensation/Korrektur des Winkelversatzes auf der Grundlage der von der Messvorrichtung (31) durchgeführten Magnetfeldmessungen;

wobei das Erzeugen grober Schätzungen der Position und des Weges des Benutzers beinhaltet:

- Bestimmung der Tritteigenschaften des Benutzers auf der Grundlage der von der Messvorrichtung (31) durchgeführten Inertialmessungen; und
- Erzeugen der groben Schätzungen von Position und Weg des Benutzers auf der Grundlage der von der Messvorrichtung (31) durchgeführten Inertialmessungen und der bestimmten Tritteigenschaften des Benutzers;

wobei das Bestimmen der Tritteigenschaften des Benutzers das Erfassen von Haltezeitintervallen einschließt, während derer der Fuß des Benutzers zwischen aufeinanderfolgenden Schritten vollständig auf dem Boden ruht, wodurch er in allen Richtungen eine Geschwindigkeit von Null hat, wobei jedes Haltezeitintervall auf einen entsprechenden Schritt des Benutzers bezogen ist;

wobei das Erzeugen der groben Schätzungen der Position und des Weges des Benutzers das Bestimmen von Rotationsmatrizen zwischen einem ersten und einem zweiten Referenzsystem einschließt, wobei das erste Referenzsystem integral mit der Erde und das zweite Referenzsystem integral mit der Messvorrichtung (31) ist; **dadurch gekennzeichnet, dass** die Verfeinerung der groben Schätzungen der Position und des Weges des Benutzers folgendes umfasst:

- für jeden Schritt des Benutzers, Speichern der jeweiligen Durchschnittswerte von

- den von der Messvorrichtung (31) im entsprechenden Haltezeitintervall durchgeführten Magnetfeldmessungen, welche sich auf den Schritt des Benutzers beziehen, und
- den Rotationsmatrizen, welche für das entsprechende Haltezeitintervall in Bezug auf den Schritt des Benutzers bestimmt wurden;

- Definieren von magnetischen Kalibrierungsmetriken, welche sich auf die letzten N Schritte des Benutzers beziehen, wobei N eine ganze Zahl größer als eins ist, und wobei die magnetischen Kalibrierungsmetriken so definiert sind, dass

-- kalibrierte Magnetfeldwerte im zweiten Bezugssystem, welche sich auf die letzten N Schritte des Benutzers beziehen, auf der Grundlage der Durchschnittswerte der für die letzten N Schritte des Benutzers gespeicherten Magnetfeldmessungen und der magnetischen Kalibrierungsparameter, welche sich auf die letzten N Schritte des Benutzers beziehen, definiert werden,
- kalibrierte Magnetfeldwerte im ersten Bezugssystem, welche sich auf die letzten N Schritte des Benutzers beziehen, auf der Grundlage der Durchschnittswerte der für die letzten N Schritte des Benutzers gespeicherten Rotationsmatrizen und der kalibrierten Magnetfeldwerte im zweiten Bezugssystem, welche sich auf die letzten N Schritte des Benutzers beziehen, definiert werden,
- kalibrierte magnetische Kurswinkel, welche sich auf die letzten N Schritte des Benutzers beziehen, auf der Grundlage der kalibrierten Magnetfeldwerte im ersten Bezugssystem, welche sich auf die letzten N Schritte des Benutzers beziehen, definiert werden, und
- eine Differenzfunktion, welche eine Differenz zwischen dem magnetischen Kurs und einen entsprechenden Winkelversatz angibt, definiert wird;

- Schätzung der magnetischen Kalibrierungsparameter, welche sich auf die letzten N Schritte des Benutzers beziehen, durch Anwendung einer vordefinierten multivariaten Minimierungstechnik auf die magnetischen Kalibrierungsmetriken, welche sich auf die letzten N Schritte des Benutzers beziehen, um so Werte der magnetischen Kalibrierungsparameter zu bestimmen, welche eine Varianz der Differenzfunktion minimieren;
- Schätzung von Werten des Winkelversatzes und der Orientierung in Bezug auf den magnetischen Norden für die letzten N Schritte des Benutzers auf der Grundlage der geschätzten Werte der magnetischen Kalibrierungsparameter, welche sich auf die letzten N Schritte des Benutzers beziehen; und
- Korrektur der groben Schätzungen der Position und des Weges des Benutzers für die letzten N Schritte des Benutzers auf der Grundlage der für die letzten N Schritte des Benutzers geschätzten Werte des Winkelversatzes und der Orientierung in Bezug auf den magnetischen Norden.

2. Inertialpositionierungs- und -verfolgungsverfahren nach Anspruch 1, wobei sich die kalibrierten Magnetfeldwerte im zweiten Referenzsystem, welche sich auf die letzten N Schritte des Benutzers beziehen, von den Durchschnittswerten der Magnetfeldmessungen, welche für die letzten N Schritte des Benutzers gespeichert wurden, durch die magnetischen Kalibrierungsparameter unterscheiden, welche konstante additive Koeffizienten sind.

**3.** Inertialpositionierungs- und -verfolgungsverfahren nach Anspruch 1 oder 2, wobei die Schritte des Definierens von magnetischen Kalibrierungsmetriken, welche sich auf die letzten N Schritte des Benutzers beziehen, der Schätzung der magnetischen Kalibrierungsparameter, welche sich auf die letzten N Schritte des Benutzers beziehen, der Schätzung von Werten des Winkelversatzes und der Orientierung in Bezug auf den magnetischen Norden für die letzten N Schritte des Benutzers und des Korrigierens der groben Schätzungen der Position und des Weges des Benutzers für die letzten N Schritte des Benutzers alle P Schritte des Benutzers ausgeführt werden, wobei P eine ganze Zahl größer als Null und kleiner als N ist.

**4.** Inertialpositionierungs- und -verfolgungssystem (3), umfassend:

- eine Messvorrichtung (31), welche so gestaltet ist, dass sie an einem Fuß eines zu ortenden und zu verfolgenden Benutzers befestigt werden kann, und welche so konfiguriert ist, dass sie Inertialmessungen und Magnetfeldmessungen durchführt, wobei die Inertialmessungen Beschleunigungsmessungen und Lageänderungsmessungen umfassen; und
- Verarbeitungsmittel (32), welche so konfiguriert sind, dass sie Signale/Daten empfangen, welche die von der Messvorrichtung (31) durchgeführten Inertialmessungen und Magnetfeldmessungen anzeigen, und dass sie die Schritte des Erzeugens von groben Schätzungen der Position und des Weges des Benutzers und des Verfeinerns der groben Schätzungen der Position und des Weges des Benutzers des Inertialpositionierungs- und -verfolgungsverfahrens, wie in einem der vorhergehenden Ansprüche definiert, ausführen.

**5.** Inertialpositionierungs- und -verfolgungssystem nach Anspruch 4, wobei die Verarbeitungsmittel in eine tragbare Verarbeitungsvorrichtung (32) integriert sind, welche:

- dazu ausgebildet ist, vom Benutzer transportiert zu werden, um geortet und verfolgt zu werden, wenn der Benutzer die Messvorrichtung (31) an seinem Fuß trägt; und
- so konfiguriert ist, dass es auf drahtlose oder verdrahtete Weise mit der Messvorrichtung (31) verbunden wird, um Signale/Daten zu empfangen, welche die von der Messvorrichtung (31) durchgeführten Inertialmessungen und Magnetfeldmessungen anzeigen.

**6.** Inertialpositionierungs- und -verfolgungssystem nach Anspruch 5, wobei das tragbare Verarbeitungsgerät (32) ein Smartphone, ein Tablet oder ein tragbares elektronisches Taschengerät ist.

**7.** Inertialpositionierungs- und -verfolgungssystem nach Anspruch 4, wobei die Messvorrichtung (31) und die Verarbeitungsmittel (32) in ein und dasselbe Gerät integriert sind.

**8.** Inertialpositionierungs- und -verfolgungssystem nach einem der Ansprüche 4-7, welches ferner eine Fernsteuerungs- und Nachbearbeitungsstation (33) umfasst, welche so konfiguriert ist, dass sie:

- Signale/Daten empfängt, welche die verfeinerten Schätzungen der Position und des Weges des Benutzers anzeigen, welche von den Verarbeitungsmitteln (32) erzeugt wurden; und
- eine weitere Verfeinerung der verfeinerten Schätzungen der Position und des Wegs des Benutzers durch eine oder mehrere vordefinierte Nachbearbeitungstechniken durchführt.

**9.** Computerprogrammprodukt, welches einen oder mehrere Teile eines Software- und/oder Firmware-Codes umfasst, welcher auf Verarbeitungsmitteln (32) eines Inertialpositionierungs- und - verfolgungssystems (3) geladen werden kann; wobei das Inertialpositionierungs- und -verfolgungssystem (3) auch eine Messvorrichtung (31) umfasst, welche dafür ausgelegt ist, an einem Fuß eines zu ortenden und zu verfolgenden Benutzers befestigt zu werden, und so konfiguriert ist, dass sie Inertialmessungen und Magnetfeldmessungen durchführt, wobei die Inertialmessungen Beschleunigungsmessungen und Lageänderungsmessungen umfassen;

wobei die Verarbeitungsmittel (32) so konfiguriert sind, dass sie Signale/Daten empfangen, welche die von der Messvorrichtung (31) durchgeführten Inertialmessungen und Magnetfeldmessungen anzeigen, und wobei der (die) Teil(e) des Software- und/oder Firmware-Codes derart ausgelegt ist (sind), dass er (sie), wenn er (sie) auf die Verarbeitungsmittel (32) geladen ist (sind), bewirkt (bewirken), dass letztere so konfiguriert werden, dass sie die Schritte des Erzeugens grober Schätzungen der Position und des Weges des Benutzers und des Verfeinerns der groben Schätzungen der Position und des Weges des Benutzers des Inertialpositionierungs- und -verfolgungsverfahrens, wie in einem der Ansprüche 1 bis 3 definiert, ausführen.

**Revendications**

1. Procédé de localisation et suivi inertiels, comprenant la fixation, sur un pied d'un utilisateur à localiser et suivre, d'un dispositif de mesure (31) configuré pour réaliser des mesures inertielles et des mesures de champ magnétique, les mesures inertielles comportant des mesures d'accélération et des mesures de variation d'attitude ;

le procédé de localisation et suivi inertiels comprenant en outre la réalisation des opérations suivantes par des moyens de traitement (32) :

• génération d'estimations grossières de position et de trajectoire de l'utilisateur sur la base des mesures inertielles réalisées par le dispositif de mesure (31) ; et
• affinement des estimations grossières de position et de trajectoire de l'utilisateur par réalisation d'une compensation/correction de dérive angulaire sur la base des mesures de champ magnétique réalisées par le dispositif de mesure (31) ;

dans lequel la génération d'estimations grossières de position et de trajectoire de l'utilisateur comporte :

• la détermination de caractéristiques de pas de l'utilisateur sur la base des mesures inertielles réalisées par le dispositif de mesure (31) ; et
• la génération des estimations grossières de position et de trajectoire de l'utilisateur sur la base des mesures inertielles réalisées par le dispositif de mesure (31) et des caractéristiques de pas de l'utilisateur déterminées ;

dans lequel la détermination de caractéristiques de pas de l'utilisateur comporte la détection d'intervalles de temps d'arrêt pendant lesquels le pied de l'utilisateur repose entièrement sur le sol entre pas successifs, ayant ainsi une vitesse nulle dans toutes les directions, chaque intervalle de temps d'arrêt étant associé à un pas correspondant de l'utilisateur ;
dans lequel la génération des estimations grossières de position et de trajectoire de l'utilisateur comporte la détermination de matrices de rotation entre un premier et un deuxième système de référence, ledit premier système de référence étant solidaire de la Terre et ledit deuxième système de référence étant solidaire du dispositif de mesure (31) ;
**caractérisé en ce que** l'affinement des estimations grossières de position et de trajectoire de l'utilisateur comporte :

• pour chaque pas de l'utilisateur, le stockage de valeurs moyennes respectives

- des mesures de champ magnétique réalisées par le dispositif de mesure (31) dans l'intervalle de temps d'arrêt correspondant associé audit pas de l'utilisateur, et
- des matrices de rotation déterminées pour l'intervalle de temps d'arrêt correspondant associé audit pas de l'utilisateur ;

• la définition de métriques d'étalonnage magnétique associées aux N derniers pas de l'utilisateur, N étant un entier supérieur à un, et lesdites métriques d'étalonnage magnétique étant définies de telle sorte que

- des valeurs de champ magnétique étalonnées dans le deuxième système de référence associées aux N derniers pas de l'utilisateur sont définies sur la base des valeurs moyennes des mesures de champ magnétique stockées pour lesdits N derniers pas de l'utilisateur et de paramètres d'étalonnage magnétique associés auxdits N derniers pas de l'utilisateur,
- des valeurs de champ magnétique étalonnées dans le premier système de référence associées aux N derniers pas de l'utilisateur sont définies sur la base des valeurs moyennes des matrices de rotation stockées pour lesdits N derniers pas de l'utilisateur et des valeurs de champ magnétique étalonnées dans le deuxième système de référence associées auxdits N derniers pas de l'utilisateur,
- des angles de cap magnétique étalonnés associés aux N derniers pas de l'utilisateur sont définis sur la base des valeurs de champ magnétique étalonnées dans le premier système de référence associées auxdits N derniers pas de l'utilisateur, et
- une fonction de différence est définie, laquelle est indicative d'une différence entre un cap magnétique et une dérive angulaire correspondante ;

- l'estimation des paramètres d'étalonnage magnétique associés aux N derniers pas de l'utilisateur par application d'une technique de minimisation multivariée prédéfinie aux métriques d'étalonnage magnétique associées auxdits N derniers pas de l'utilisateur de manière à déterminer des valeurs desdits paramètres d'étalonnage magnétique qui minimisent une variance de la fonction de différence ;
- l'estimation de valeurs de dérive angulaire et d'orientation par rapport au Nord magnétique pour les N derniers pas de l'utilisateur sur la base des valeurs estimées des paramètres d'étalonnage magnétique associés auxdits N derniers pas de l'utilisateur ; et
- la correction des estimations grossières de position et de trajectoire de l'utilisateur pour les N derniers pas dudit utilisateur sur la base des valeurs de dérive angulaire et d'orientation par rapport au Nord magnétique estimées pour lesdits N derniers pas de l'utilisateur.

**2.** Procédé de localisation et suivi inertiels selon la revendication 1, dans lequel les valeurs de champ magnétique étalonnées dans le deuxième système de référence associées aux N derniers pas de l'utilisateur diffèrent des valeurs moyennes des mesures de champ magnétique stockées pour lesdits N derniers pas de l'utilisateur par les paramètres d'étalonnage magnétique, qui sont des coefficients additifs constants.

**3.** Procédé de localisation et suivi inertiels selon la revendication 1 ou 2, dans lequel les étapes de définition de métriques d'étalonnage magnétique associées aux N derniers pas de l'utilisateur, estimation des paramètres d'étalonnage magnétique associés aux N derniers pas de l'utilisateur, estimation de valeurs de dérive angulaire et d'orientation par rapport au Nord magnétique pour les N derniers pas de l'utilisateur, et correction des estimations grossières de position et de trajectoire de l'utilisateur pour les N derniers pas dudit utilisateur sont réalisées tous les P pas de l'utilisateur, P étant un entier supérieur à zéro et inférieur à N.

**4.** Système de localisation et suivi inertiels (3), comprenant :

- un dispositif de mesure (31), qui est destiné à être fixé sur un pied d'un utilisateur à localiser et suivre, et est configuré pour réaliser des mesures inertielles et des mesures de champ magnétique, les mesures inertielles comportant des mesures d'accélération et des mesures de variation d'attitude ; et
- des moyens de traitement (32) configurés pour recevoir des signaux/données indicatifs des mesures inertielles et des mesures de champ magnétique réalisées par le dispositif de mesure (31) et pour réaliser les étapes de génération d'estimations grossières de position et de trajectoire de l'utilisateur et d'affinement des estimations grossières de position et de trajectoire de l'utilisateur du procédé de localisation et suivi inertiels tel que défini dans l'une quelconque des revendications précédentes.

**5.** Système de localisation et suivi inertiels selon la revendication 4, dans lequel les moyens de traitement sont intégrés dans un dispositif de traitement portable (32), qui est :

- destiné à être transporté par l'utilisateur à localiser et suivre quand ledit utilisateur porte le dispositif de mesure (31) sur son pied ; et
- configuré pour être raccordé, sans fil ou de façon filaire, audit dispositif de mesure (31) pour recevoir des signaux/données indicatifs des mesures inertielles et des mesures de champ magnétique réalisées par le dispositif de mesure (31).

**6.** Système de localisation et suivi inertiels selon la revendication 5, dans lequel le dispositif de traitement portable (32) est un mobile multifonction, une tablette, ou un dispositif électronique portable, de poche.

**7.** Système de localisation et suivi inertiels selon la revendication 4, dans lequel le dispositif de mesure (31) et les moyens de traitement (32) sont intégrés dans un seul et même dispositif.

**8.** Système de localisation et suivi inertiels selon l'une quelconque des revendications 4 à 7, comprenant en outre une station de commande à distance et de post-traitement (33) configurée pour :

- recevoir des signaux/données indicatifs des estimations affinées de position et de trajectoire de l'utilisateur générées par les moyens de traitement (32) ; et
- réaliser un affinement supplémentaire des estimations affinées de position et de trajectoire de l'utilisateur par le biais d'une ou plusieurs techniques de post-traitement prédéfinies.

**9.** Produit programme d'ordinateur comprenant une ou plusieurs parties de code de programme et/ou de microprogramme chargeables sur des moyens de traitement (32) d'un système de localisation et suivi inertiels (3), ledit système de localisation et suivi inertiels (3) comprenant également un dispositif de mesure (31), qui est destiné à être fixé sur un pied d'un utilisateur à localiser et suivre, et est configuré pour réaliser des mesures inertielles et des mesures de champ magnétique, les mesures inertielles comportant des mesures d'accélération et des mesures de variation d'attitude ;

les moyens de traitement (32) étant configurés pour recevoir des signaux/données indicatifs des mesures inertielles et des mesures de champ magnétique réalisées par le dispositif de mesure (31), et ladite/lesdites partie(s) de code de programme et/ou de microprogramme est/sont telle(s) qu'elle(s) condui(sen)t, lorsqu'elle(s) est/sont chargée(s) sur les moyens de traitement (32), ces derniers à être configurés pour réaliser les étapes de génération d'estimations grossières de position et de trajectoire de l'utilisateur et d'affinement des estimations grossières de position et de trajectoire de l'utilisateur du procédé de localisation et suivi inertiels tel que défini dans l'une quelconque des revendications 1 à 3.

Fig. 1

Fig. 2

Fig. 3

Number of steps

Fig. 4

Fig. 5

Fig. 6

Fig. 7

Fig. 8

Fig. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- IT 102017000119916
- EP 2345293 B1
- US 20170026804 A1
- IT RM20120641 A
- IT RM2012 A
- IT 000641 A
- US 2014203970 A1
- IT 102017000088521

### Non-patent literature cited in the description

- **YAN XIA LIU ; XI SHENG LI ; XIAO JUAN ZHANG ; YI BO FENG.** Novel Calibration Algorithm for a Three-Axis Strapdown Magnetometer. *Sensors,* 2014, vol. 14 (5), 8485-8504
- **VITALI ANDREA.** Ellipsoid or sphere fitting for sensor calibration. *ST Microelectronics, DT0059 Design Tip, DT 0059 Rev,* 02 August 2016
- **M. BAZARAA ; H. SHERALI ; C. SHETTY.** Nonlinear Programming, Theory and Applications. J. Wiley & Sons, New York, 2006